# EUROPÄISCHE PATENTSCHRIFT

(11) **EP 1 279 371 B1**
(45) Veröffentlichungstag und Bekanntmachung des Hinweises auf die Patenterteilung: **22.12.2004**
(21) Anmeldenummer: 02012519.1
(22) Anmeldetag: 05.06.2002
(51) Int. Cl.: A61B 17/28, A61B 17/32

(54) **Chirurgisches Instrument**
Surgical instrument
Instrument chirurgical

(30) Priorität: 28.07.2001 DE 10136964
(43) Veröffentlichungstag der Anmeldung: 29.01.2003
(73) Patentinhaber: Aesculap AG & Co. KG, 78532 Tuttlingen (DE)
(72) Erfinder: Mayenberger, Rupert, 78239 Rielasingen (DE); Schweitzer, Tom, 78532 Tuttlingen-Nendingen (DE)
(74) Vertreter: HOEGER, STELLRECHT & PARTNER Patentanwälte

(56) Entgegenhaltungen:
- US-A- 2 518 994
- US-A- 5 290 309
- US-A- 5 820 009
- US-A- 5 989 277
- US-A- 6 019 780

## Beschreibung

Die Erfindung betrifft ein chirurgisches Instrument mit einem Schaft, mit einem ersten, am distalen Ende des Schafts angeordneten, ein um eine Schwenkachse schwenkbar gelagertes Werkzeugelement umfassenden Werkzeug und mit einem ersten Kraftübertragungsglied zum Verschwenken des ersten Werkzeugelements von einer ersten Schwenkstellung in eine zweite Schwenkstellung, wobei eine erste Steuerkurve zur Transformation einer Bewegung des ersten Kraftübertragungsglieds in die Schwenkbewegung des ersten Werkzeugelements vorgesehen ist und wobei die Steuerkurve eine Form aufweist zum Umwandeln einer von dem ersten Kraftübertragungsglied auf das erste Werkzeugelement ausübbaren Kraft in ein bei der Schwenkbewegung zunehmendes Drehmoment, wobei die erste Steuerkurve zwei aneinander entlanggleitende Steuerflächen umfaßt und wobei die eine der beiden Steuerflächen eine Stirnkante des ersten Kraftübertragungsglieds und die andere dem ernsten Werkzeugelement zugeordnet ist.

Chirurgische Instrumente der eingangs beschriebenen Art sind zum Beispiel aus der US 2,518,994 bekannt und werden beispielsweise bei chirurgischen Eingriffen am menschlichen Körper verwendet, insbesondere bei endoskopischen Eingriffen. Die Instrumente sind beispielsweise als Zangen, Scheren oder dergleichen ausgestaltet. US 2,518,994 offenbart ein chirurgisches Instrument gemäß dem Oberbegriff des Anspruchs 1.

Im Gegensatz zu herkömmlichen Scheren oder Zangen wird eine Kraft von einem meist als Schub- und Zugglied ausgestalteten Betätigungsglied indirekt auf das Werkzeug übertragen. Dies ist insbesondere dann nachteilig, wenn das Werkzeug zum Fassen oder Schneiden verwendet wird und sich im wesentlichen in der Faß- oder Schneidstellung befindet, denn bei herkömmlichen Instrumenten ist eine Schließkraft in diesen Stellungen stets am geringsten.

Daher ist es Aufgabe der vorliegenden Erfindung, ein Instrument der eingangs beschriebenen Art so zu verbessern, daß das erste Werkzeugelement leichter von der zweiten in die erste Werkzeugelementstellung verschwenkt werden kann.

Diese Aufgabe wird bei einem chirurgischen Instrument der eingangs beschriebenen Art erfindungsgemäß dadurch gelöst, daß ein zweites Kraftübertragungsglied zum Rückverschwenken des ersten Werkzeugelements von der zweiten Schwenkstellung in die erste Schwenkstellung vorgesehen ist, daß eine zweite Steuerkurve zur Transformation einer Bewegung des zweiten Kraftübertragungsglieds in eine Schwenkbewegung des ersten Werkzeugelements von der zweiten Werkzeugelementstellung in die erste Werkzeugelementstellung vorgesehen ist und daß die Steuerkurve eine Form aufweist zum Umwandeln einer von dem zweiten Kraftübertragungsglied auf das erste Werkzeugelement ausübbaren Kraft in ein bei der Schwenkbewegung zunehmendes Drehmoment.

Durch diese besonders geformte Steuerkurve nimmt die von dem ersten Werkzeugelement ausübbare Kraft bei einer Verschwenkung von der ersten Schwenkstellung in die zweite Schwenkstellung zu, egal in welche Richtung die Schwenkbewegung ausgeführt wird. Damit lassen sich, insbesondere bei zangen- oder scherenartigen Instrumenten, auch noch in extremen Schnittoder Faßstellungen größere Kräfte als bisher übertragen. Dies hat insbesondere den Vorteil, daß bei einer Ausgestaltung der Kraftübertragungsglieder als reine Schubglieder auf ein üblicherweise erforderliches Koppelelement zwischen dem Kraftübertragungsglied und dem ersten Werkzeugelement verzichtet werden kann. Darüber hinaus lassen sich derartige Flächen besonders einfach herstellen. Durch das zweite Kraftübertragungsgerad läßt sich die Schwenkbewegung in die eine Richtung von der Schwenkbewegung in die andere Richtung entkoppeln. Beispielsweise können beide Kraftübertragungsglieder nur Zug- oder nur Schubkräfte auf das erste Werkzeugelement übertragen, was bei herkömmlichen Instrumenten, die eine Schub- und Zugstange aufweisen, nicht möglich ist. Bei einem Instrumen, welches eine zweite steuerkurve aufweist die mit dem ersten Werkzeugelement ausübbare Kraft stets zu, egal in welche Richtung es verschwenkt wird. Dies hat sowohl bei einem Verschwenken des Werkzeugelements, beispielsweise zum Öffnen oder aber auch zum Schließen einer Zange, Vorteile, insbesondere jedoch beim Schließen, so daß bei Scheren ein sauberer Schnitt bis zum Ende der Schwenkbewegung von aneinander abgleitenden Schneiden ermöglicht wird.

Vorzugsweise weist die Steuerkurve eine Form auf zum Verlängern des effektiven, am ersten Werkzeugelement wirkenden Hebelarms bei der Schwenkbewegung von der ersten Schwenkstellung in die zweite Schwenkstellung. Damit lassen sich beispielsweise bei konstanter, vom Kraftübertragungsglied auf das erste Werkzeugelement übertragener Kraft, größere Drehmomente erzeugen und damit die von dem ersten Werkzeugelement ausübbare Kraft erhöhen.

Vorteilhaft ist es, wenn die Steuerflächen zwei im wesentlichen ebene Flächen umfassen und wenn die dem Kraftübertragungsglied zugeordnete Steuerfläche von der Schwenkachse in Richtung auf das proximale Ende des Instruments geneigt ist. Bei einer derartigen Ausgestaltung kann die dem ersten Werkzeugelement zugeordnete Steuerfläche an der dem ersten Kraftübertragungsglied zugeordneten Steuerfläche aufgleiten, wobei das übertragbare Drehmoment aufgrund eines zunehmenden Hebelarms, der im wesentlichen dem projizierten Abstand von der Drehachse entspricht, zunimmt.

Bei einer besonders bevorzugten Ausführungsform der Erfindung kann vorgesehen sein, daß die Steuerflächen während der Bewegung des ersten Werkzeugelements von der ersten Werkzeugelementstellung in die zweite Werkzeugelementstellung aneinander anliegen. Damit läßt sich eine spielfreie Verschwenkung des ersten Werkzeugelements realisieren. Ferner lassen sich bei entsprechender Gestaltung der Oberflächen Reibungsverluste minimieren.

Vorzugsweise liegen die Steuerflächen in einem ersten Berühr- oder Angriffspunkt aneinander an. Durch diese Ausgestaltung läßt sich die Reibung zwischen den beiden aneinander anliegenden Oberflächen minimieren.

Vorzugsweise umfaßt die zweite Steuerkurve zwei aneinander entlanggleitende Rückbewegungssteuerflächen und ist die eine der beiden Rückbewegungssteuerflächen dem Kraftübertragungsglied und die andere dem ersten Werkzeugelement zugeordnet. Dies ermöglicht die Herstellung besonders einfach ausgestalteter Rückbewegungssteuerflächen und einen Verzicht auf ein Koppelelement, wenn die Kraftübertragungsglieder als reine Schubglieder ausgestaltet sind.

Bei einer bevorzugten Ausführungsform der Erfindung kann vorgesehen sein, daß die dem zweiten Kraftübertragungsglied zugeordnete Steuerfläche bezogen auf die Steuerachse konkav gekrümmt ist und daß die dem ersten Werkzeugelement zugeordnete Steuerfläche bezogen auf die Schwenkachse konvex gekrümmt ist. Damit lassen sich auf einfache Weise bei der Schwenkbewegung zunehmende Drehmomente realisieren. Darüber hinaus ist in Verbindung mit derartigen Steuerflächen eine Selbsthemmung aneinander entlanggleitender Flächen unmöglich.

Vorteilhaft ist es, wenn die Rückbewegungssteuerflächen während der Bewegung des ersten Werkzeugelements von der zweiten Werkzeugelementstellung in die erste Werkzeugelementstellung aneinander anliegen. So läßt sich eine spielfreie Schwenkbewegung realisieren, wenn die Rückbewegungssteuerflächen während der gesamten Bewegung aneinander anliegen.

Vorzugsweise liegen die beiden Rückbewegungssteuerflächen an einem zweiten Berühr- oder Angriffspunkt aneinander an. Auf diese Weise lassen sich Reibungsverluste beim Bewegen des ersten Werkzeugelements minimieren.

Bei einer weiteren bevorzugten Ausführungsform der Erfindung kann vorgesehen sein, daß der erste Angriffspunkt und der zweite Angriffspunkt unabhängig von einer Schwenkstellung auf der gleichen Seite einer die Drehachse enthaltenden Ebene liegen. Durch diese Ausgestaltung lassen sich besonders lange effektive Hebelarme erzielen, da die Lagerung des ersten, schwenkbar gelagerten Werkzeugelements unsymmetrisch am Schaft realisiert werden kann. Damit steht im wesentlichen nahezu die ganze Erstreckung des Schafts quer zu seiner Längsrichtung in extremen Stellungen als effektiver Hebelarm zur Verfügung.

Zur Erhöhung der Stabilität des Instruments kann es vorteilhaft sein, wenn das erste und das zweite Kraftübertragungsglied jeweils ein Schubglied umfassen zum Übertragen einer Schubkraft auf das erste Werkzeugelement. Auf diese Weise kann auf ein Koppelelement zwischen dem ersten Werkzeugelement und dem Kraftübertragungsgliedern verzichtet werden, so daß Festigkeitsprobleme des Koppelelements völlig unbeachtlich sind.

Günstig ist es, wenn die Kraftübertragungsglieder in Längsrichtung des Schafts bewegbar sind. Damit eignet sich das Instrument insbesondere für endoskopische Eingriffe, denn der Schaft kann so mit einem minimalen Querschnitt ausgebildet werden.

Um eine Bedienung des Instruments zu vereinfachen, ist es von Vorteil, wenn die Kraftübertragungsglieder zum Bewegen der ersten Werkzeugelements gegenläufig bewegbar sind. Dies ermöglicht insbesondere auch ein automatisches Zurückziehen des einen Kraftübertragungsglieds, wenn das andere vorgeschoben wird. Die Bedienung des Instruments wird dadurch wesentlich vereinfacht.

Bei einer bevorzugten Ausführungsform der Erfindung kann vorgesehen sein, daß mindestens ein dem Schaft zugeordnetes, beweglich gelagertes Betätigungsglied vorgesehen ist zum Bewegen mindestens eines der beiden Kraftübertragungsglieder und daß das mindestens eine der beiden Kraftübertragungsglieder an dem mindestens einen Betätigungsglied gelagert ist. Diese Ausgestaltung erleichtert die Bedienung des Instruments, denn die Kraftübertragungsglieder müssen nicht direkt bewegt werden, sondern können beispielsweise über ein Betätigungsglied mit günstigeren Hebelverhältnissen besonders einfach bewegt werden.

Vorzugsweise ist das Betätigungsglied am Instrument schwenkbar gelagert. Dies vereinfacht den Konstruktionsaufwand des Instruments, denn es ist lediglich ein Lager zur Lagerung des Betätigungsglieds am Instrument erforderlich.

Vorzugsweise ist mindestens ein Anschlag zur Begrenzung der Bewegung des ersten und/oder des zweiten Kraftübertragungsglieds und/oder des ersten Werkzeugelements vorgesehen. Mit einem solchen Anschlag lassen sich extreme Schwenkstellungen des ersten Werkzeugelements genau festlegen.

Zur Vereinfachung des Aufbaus des Instruments kann in vorteilhafter Weise vorgesehen sein, daß das Werkzeug ein zweites, relativ zum Schaft feststehendes Werkzeugelement umfaßt.

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Ansicht eines erfindungsgemäßen chirurgischen Instruments;
- Figur 2:: eine Längsschnittansicht längs Linie 3-3 in Figur 4 eines Instruments während des Öffnens des Werkzeugs;
- Figur 3:: eine Längsschnittansicht längs Linie 3-3 aus Figur 4 beim Schließen des Werkzeugs;
- Figur 4:: eine Draufsicht auf das Werkzeug in Richtung des Pfeils A aus Figur 3;
- Figur 5:: eine Ansicht ähnlich Figur 2 mit einem beim Öffnen des Werkzeugs maximalen Hebelarm; und
- Figur 6:: eine Ansicht ähnlich Figur 3 mit einem beim Schließen des Werkzeugs maximalen Hebelarm.

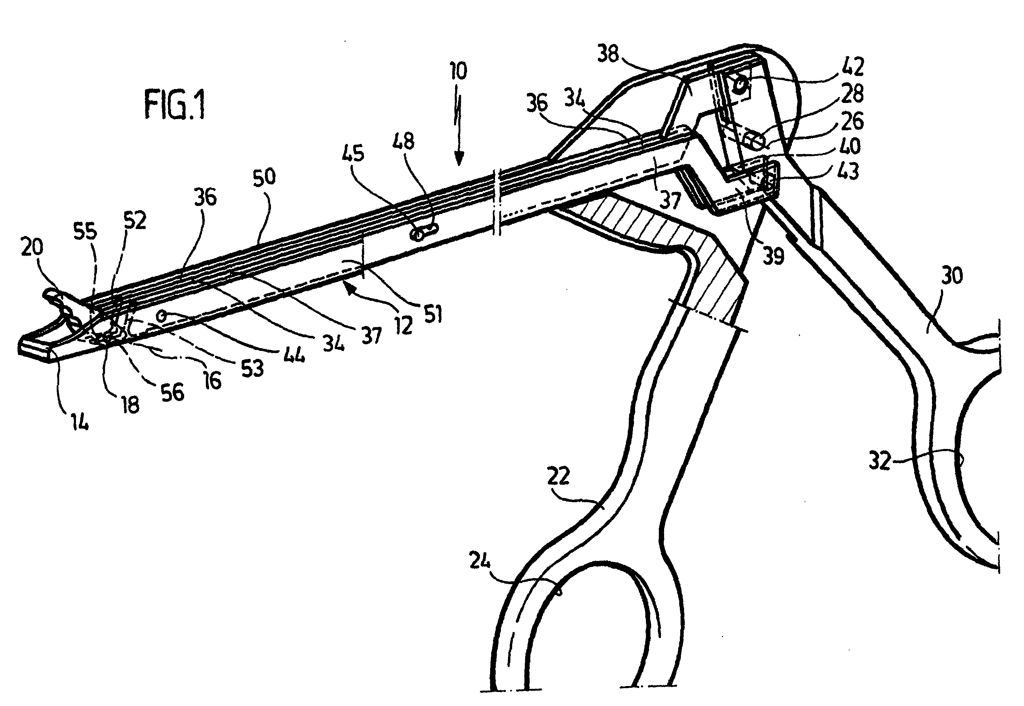

In Figur 1 ist eine insgesamt mit dem Bezugszeichen 10 versehene endoskopische Zange 10 dargestellt, mit einem eine Längsrichtung definierenden, im Querschnitt U-förmigen, einen Schaftboden 13 aufweisenden Schaft 12, an dessen distalem Ende eine in Längsrichtung abstehende bügelförmige Backe 14 angeordnet ist, sowie einer um eine quer zur Längsachse orientierte Drehachse 16 um eine Welle 18 verschwenkbar gelagerte zweite Backe 20. Ferner ist eine mit dem Schaft 12 starr verbundene erste Branche 22 vorgesehen, die an ihrem freien Ende eine Fingeröffnung 24 aufweist sowie eine im proximalen Bereich des Schafts um einen als Welle dienenden und eine Drehachse 26 definierenden Gelenkstift 28 verschwenkbare Betätigungsbranche 30, die an ihrem freien Ende ebenfalls eine Fingeröffnung 32 aufweist.

Weiterhin sind drei Kraftübertragungsglieder vorgesehen, von denen eines als öffnendes Schubglied 34 und die beiden anderen miteinander gekoppelten und parallel zum Schubglied 34 verlaufenden Schließglieder 36 und 37 als schließende Schubglieder ausgebildet sind.

An ihren proximalen Enden sind das Schubglied 34 und die beiden Schließglieder 36 und 37 voneinander weg weisend abgewinkelt und weisen parallel verlaufende Lagerabschnitte 38 beziehungsweise 39 und 40 auf, die jeweils mittels eines Lagerstifts 42 beziehungsweise 43 an der Betätigungsbranche 30 schwenkbar gelagert sind. Die Lagerstifte 42 und 43 sind im wesentlichen diametral gegenüberliegend relativ zum Gelenkstift 28 angeordnet, wobei der Abstand des Lagerstifts 42 von der Drehachse 26 dem des Lagerstifts 43 von der Drehachse 26 entspricht.

Quer zur Längsachse des Schafts 12 verlaufend sind an diesem zwei stiftförmige Führungsglieder 44 und 45 angeordnet, die einerseits sich im Schubglied 34 in Längsrichtung erstreckende langlochartige Schlitze 46, andererseits die in den beiden Schließgliedern 36 und 37 angeordneten Langlöcher 48 und 49 durchsetzen. Die Breite der Führungsglieder 44 und 45 entspricht jeweils der Breite der Schlitze 46 beziehungsweise der Langlöcher 48 und 49, so daß eine Zwangsführung des Schubglieds 34 beziehungsweise der Schließglieder 36 und 37 in Längsrichtung des Schafts 12 gebildet wird. Mit dem Schubglied 34 und den Schließgliedern 36 und 37 können mangels eines vorhandenen Koppelelements jeweils nur Schubkräfte auf die Backe 20 übertragen werden.

Die Welle 18 ist parallel zum Schaftboden 13 des U-förmigen Schafts 12 in dessen Verlängerung an Schaftseitenflächen 50 und 51 festgelegt.

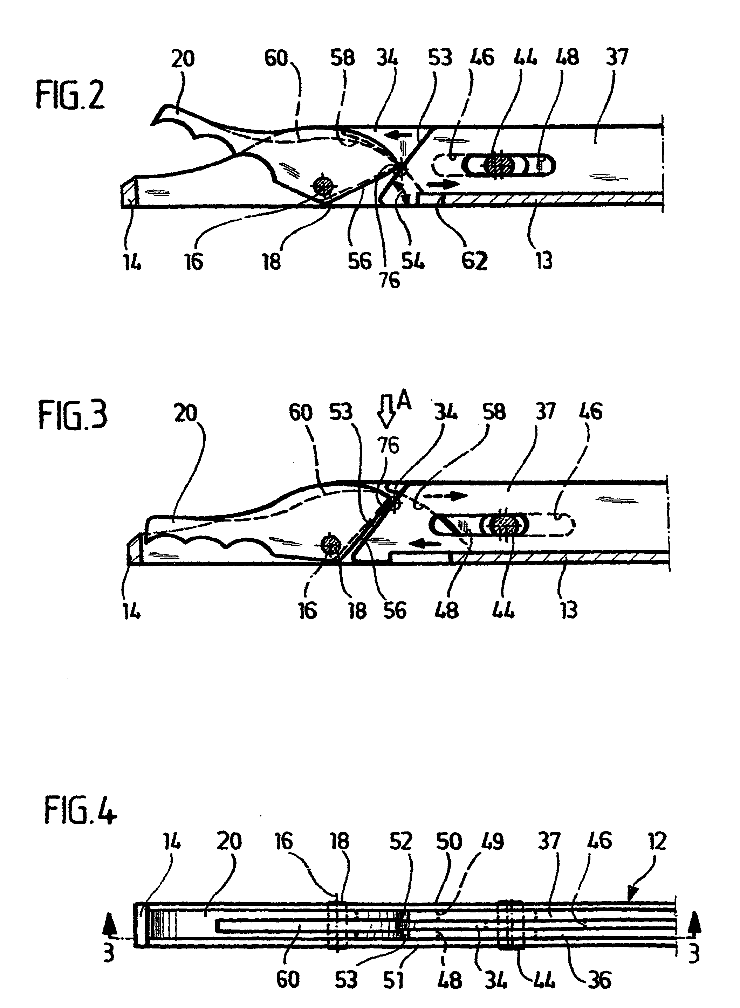

Um eine definierte Bewegung der Backe 20 zu realisieren, ist eine Stirnkante 52 des Schließglieds 36 beziehungsweise die Stirnkante 53 des Schließglieds 37 als ebene, sich unter einem Winkel 54 vom Schaftboden 13 in Richtung auf das proximale Ende des Schafts 12 hin erstreckende Flächen ausgebildet. Die Stirnkanten 52 und 53 liegen in jeder Schwenkstellung an Schließflächen 55 und 56 der Backe 20 an. In einer Schließstellung, in der sich die Schließglieder 36 und 37 in ihrer distalsten Stellung befinden, wie es im wesentlichen in Figur 3 dargestellt ist, verlaufen sie in etwa parallel zu den Stirnkanten 52 und 53. Die Stirnkanten 52 und 53 bilden zusammen mit den Schließflächen 55 und 56 eine erste, doppelt ausgebildete Steuerkurve, denn die aneinander entlanggleitenden Flächen beziehungsweise Kanten bewirken bei einer Verschiebung der Schließglieder 36 und 37 in distaler Richtung ein Schließen der Backe 20.

Zum Öffnen der Backe 20 wird diese aufgrund einer Vorschubbewegung des Schubglieds 34 verschwenkt, dessen distales Ende eine in Richtung auf die Welle 18 konkav gekrümmte Stirnfläche 58 aufweist, die an einer konvex gekrümmten, von der Welle 18 weg weisenden Steuerfläche 60 der Backe 20 anliegt. Eine Vorschubbewegung des Schubglieds 34 führt demnach zu einem Verschwenken der Backe 20, um diese zu öffnen, und zwar mittels der eine zweite Steuerkurve bildenden Stirnfläche 58 in Verbindung mit der Steuerfläche 60.

Durch die gegenüberliegende Lagerung der Schubglieder 34 beziehungsweise 36 und 37 an der Betätigungsbranche 30 führt ein Verschwenken derselben zu einer gegenläufigen Bewegung von dem Schubglied 34 beziehungsweise den Schubgliedern 36 und 37.

In jeder Schwenkstellung der Backe 20 liegen die eine erste beziehungsweise eine zweite Steuerkurve bildenden Flächen aneinander an. Die Krümmungen beziehungsweise Neigungen der Stirnfläche 58 und der Steuerfläche 60 beziehungsweise der Stirnkanten 52 und 53 und der Schließflächen 55 und 56 sind so gewählt, daß sowohl das Schubglied 34 als auch die Schließglieder 36 und 37 stets an der Backe 20 anliegen.

Durch die besondere Form der Stirnkanten 52 und 53 sowie der Stirnfläche 58 in Verbindung mit der in der Nähe des Schaftbodens 13 angeordneten Welle 18 ergibt sich ein bei konstanter, auf das Schubglied 34 beziehungsweise die Schließglieder 36 und 37 wirkender Vorschubkraft stets ein zunehmendes Drehmoment bei deren Bewegung in Richtung auf das distale Ende des Schafts hin.

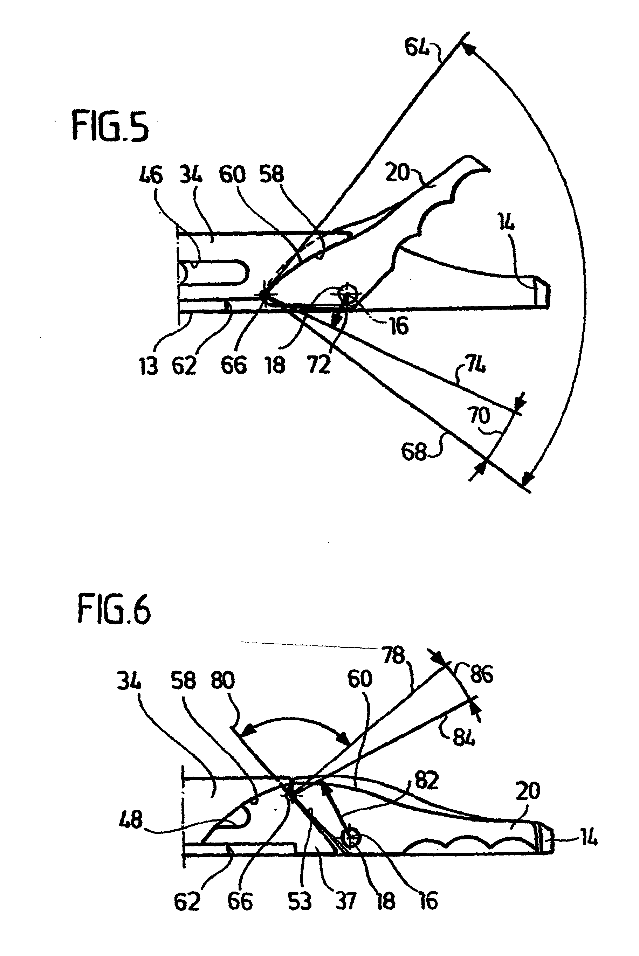

Im Zusammenhang mit Figur 5 wird die Bestimmung eines Hebelarms beim Wegverschwenken der Backe 20 von der Backe 14 erläutert.

In einem Berührpunkt 66 wird eine Tangente 64 an die Stirnfläche 58 angelegt. Eine Normale 68 zur Tangente wird eingezeichnet, wobei senkrecht zu dieser durch die Symmetrieachse der Welle 18 verlaufend ein Hebelarm einzuzeichnen wäre. Aufgrund von Reibungskräften zwischen den Oberflächen der aneinander entlanggleitenden Stirnfläche 58 und der Steuerfläche 60, ergibt sich ein effektiver Hebelarm 72 als Lot relativ zu einer Resultierenden 74, die mit der Normalen 68 einen Reibungswinkel 70 einschließt, der von der jeweiligen Reibungszahl der verwendeten Materialien abhängt. Je weiter sich die Backe 20 von der Backe 14 entfernt, um so größer wird dann der effektive Hebelarm 72.

Im Zusammenhang mit Figur 6 wird die Vergrößerung eines beim Aufeinanderzubewegen der Backe 14 und der Backe 20 wirkenden Hebelarms 82 erläutert.

Wie im Zusammenhang mit Figur 5 erläutert, müßte ohne Reibung zwischen den Schließgliedern 36 und 37 sowie der Backe 20 ein Hebelarm senkrecht zu einer Normalen 78 konstruiert werden, die wiederum senkrecht zu einer Tangente 80 an dem Berührpunkt 76 zwischen der Stirnkante 52 beziehungsweise 53 und der Schließfläche 55 beziehungsweise 56 angelegt wird. Aufgrund von Reibung zwischen den aneinander entlanggleitenden Materialien ist der effektive Hebelarm 82 jedoch senkrecht bezüglich einer Resultierenden 84 zu ermitteln, die mit der Normalen 78 den rechten Winkel zwischen der Normalen 78 und der Tangenten 80 um einen Reibungswinkel 86 reduziert.

Je weiter die Backe 20 von der Backe 14 entfernt ist, um so kleiner wird der effektive Hebelarm 82. Anders herum ausgedrückt: Durch Bewegung der Schließglieder 36 und 37 in distaler Richtung wird die Backe 20 in Richtung auf die Backe 14 verschwenkt, wobei der effektive Hebelarm 82 oder das wirkende Drehmoment kontinuierlich zunimmt.

Durch die besondere, anders als die beiden Führungsglieder 44 und 45 außermittige Anordnung der die Drehachse 16 bildenden Welle 18 benachbart einer Verlängerung des Schaftbodens 13 in distaler Richtung, liegen der erste Berührpunkt 66 und der zweite Berührpunkt 76 beide auf der gleichen Seite einer die Drehachse 16 enthaltenden Ebene, und zwar unabhängig von einer Schwenkstellung der zweiten Backe 20. Dies hat den Vorteil, daß die effektiven Hebelarme 72 und 82 besonders groß sein können, nämlich im Extremfall dem Abstand der Berührpunkte 66 bzw. 76 von der Drehachse 16 entsprechen, welche sogar größer sein können als die maximale Ausdehnung des Schafts 12 quer zu seiner Längsrichtung.

## Patentansprüche

1. Chirurgisches Instrument (10) mit einem Schaft (12), mit einem ersten, am distalen Ende des Schafts (12) angeordneten, ein um eine Schwenkachse (16) schwenkbar gelagertes Werkzeugelement (20) umfassenden Werkzeug und mit einem ersten Kraftübertragungsglied (36, 37) zum Verschwenken des ersten Werkzeugelements (20) von einer ersten Schwenkstellung in eine zweite Schwenkstellung, wobei eine erste Steuerkurve (52, 53; 55, 56) zur Transformation einer Bewegung des ersten Kraftübertragungsglieds (36, 37) in die Schwenkbewegung des ersten Werkzeugelements (20) vorgesehen ist und wobei die erste Steuerkurve (52, 53; 55, 56) eine Form aufweist zum Umwandeln einer von dem ersten Kraftübertragungsglied (36, 37) auf das erste Werkzeugelement (20) ausübbaren Kraft in ein bei der Schwenkbewegung zunehmendes Drehmoment, wobei die erste Steuerkurve zwei aneinander entlanggleitende Steuerflächen (52, 53; 55, 56) umfaßt und wobei die eine der beiden Steuerflächen (52, 53) eine Stirnkante (52, 53) des ersten Kraftübertragungsglieds (36, 37) und die andere (55, 56) dem ersten Werkzeugelement (20) zugeordnet ist, **dadurch gekennzeichnet, daß** ein zweites Kraftübertragungsglied (34) zum Rückverschwenken des ersten Werkzeugelements (20) von der zweiten Schwenkstellung in die erste Schwenkstellung vorgesehen ist, daß eine zweite Steuerkurve (58, 60) zur Transformation einer Bewegung des zweiten Kraftübertragungsglieds (34) in eine Schwenkbewegung des ersten Werkzeugelements (20) von der zweiten Werkzeugelementstellung in die erste Werkzeugelementstellung vorgesehen ist und daß die Steuerkurve (58, 60) eine Form aufweist zum Umwandeln einer von dem zweiten Kraftübertragungsglied (34) auf das erste Werkzeugelement (20) ausübbaren Kraft in ein bei der Schwenkbewegung zunehmendes Drehmoment.

2. Instrument nach Anspruch 1, **dadurch gekennzeichnet, daß** die erste Steuerkurve (52, 53; 55, 56) eine Form aufweist zum Verlängern des effektiven, am ersten Werkzeugelement (20) wirkenden Hebelarms (72) bei der Schwenkbewegung von der ersten Schwenkstellung in die zweite Schwenkstellung.

3. Instrument nach einem der Ansprüche 1 oder 2, **dadurch gekennzeichnet, daß** die Steuerflächen zwei im wesentlichen ebene Flächen (52, 53; 55, 56) umfassen und daß die dem ersten Kraftübertragungsglied (36, 37) zugeordnete Steuerfläche (52, 53) von der Schwenkachse in Richtung auf das proximale Ende des Instruments (10) geneigt ist.

4. Instrument nach einem der voranstehenden Ansprüche, **dadurch gekennzeichnet, daß** die Steuerfläche (52, 53; 55, 56) während der Bewegung des ersten Werkzeugelements (20) von der ersten Werkzeugelementstellung in die zweite Werkzeugelementstellung aneinander anliegen.

5. Instrument nach Anspruch 5, **dadurch gekennzeichnet, daß** die Steuerflächen (52, 53; 55, 56) in einem ersten Berühr- oder Angriffspunkt (76) aneinander anliegen.

6. Instrument nach einem der voranstehenden Ansprüche, **dadurch gekennzeichnet, daß** die zweite Steuerkurve zwei aneinander entlanggleitende Rückbewegungssteuerflächen (58, 60) umfaßt und daß die eine der beiden Rückbewegungssteuerflächen (58) dem zweiten Kraftübertragungsglied (34) und die andere (60) dem ersten Werkzeugelement (20) zugeordnet ist.

7. Instrument nach Anspruch 6, **dadurch gekennzeichnet, daß** die dem zweiten Kraftübertragungsglied (34) zugeordnete Steuerfläche (58) bezogen auf die Schwenkachse konkav gekrümmt ist und daß die dem ersten Werkzeugelement (20) zugeordnete Steuerfläche (60) bezogen auf die Schwenkachse konvex gekrümmt ist.

8. Instrument nach einem der Ansprüche 6 oder 7, **dadurch gekennzeichnet, daß** die Rückbewegungssteuerflächen (58, 60) während der Bewegung des ersten Werkzeugelements (20) von der zweiten Werkzeugelementstellung in die erste Werkzeugelementstellung aneinander anliegen.

9. Instrument nach Anspruch 8, **dadurch gekennzeichnet, daß** die Rückbewegungssteuerflächen (58, 60) in einem zweiten Berühr- oder Angriffspunkt (66) aneinander anliegen.

10. Instrument nach Anspruch 9, **dadurch gekennzeichnet, daß** der erste Angriffspunkt (76) und der zweite Angriffspunkt (66) unabhängig von einer Schwenkstellung des ersten Werkzeugelements (20) auf der gleichen Seite einer die Drehachse (16) enthaltenden Ebene liegen.

11. Instrument nach einem der voranstehenden Ansprüche, **dadurch gekennzeichnet, daß** das erste (36, 37) und das zweite Kraftübertragungsglied (34) jeweils ein Schubglied (36, 37; 34) umfassen zum Übertragen einer Schubkraft auf das erste Werkzeugelement (20).

12. Instrument nach einem der voranstehenden Ansprüche, **dadurch gekennzeichnet, daß** die Kraftübertragungsglieder (36, 37; 34) in Längsrichtung des Schafts (12) bewegbar sind.

13. Instrument nach einem der voranstehenden Ansprüche, **dadurch gekennzeichnet, daß** die Kraftübertragungsglieder (36, 37; 34) zum Bewegen des ersten Werkzeugelements (20) gegenläufig bewegbar sind.

14. Instrument nach einem der voranstehenden Ansprüche, **dadurch gekennzeichnet, daß** mindestens ein dem Schaft (12) zugeordnetes, beweglich gelagertes Betätigungsglied (30) vorgesehen ist zum Bewegen mindestens eines der beiden Kraftübertragungsglieder (36, 37; 34) und daß das mindestens eine der beiden Kraftübertragungsglieder (36, 37; 34) an dem mindestens einen Betätigungsglied (30) gelagert ist.

15. Instrument nach Anspruch 14, **dadurch gekennzeichnet, daß** das Betätigungsglied (30) am Instrument (10) schwenkbar gelagert ist.

16. Instrument nach einem der voranstehenden Ansprüche, **dadurch gekennzeichnet, daß** mindestens ein Anschlag (62) zur Begrenzung der Bewegung des ersten (36, 37) und/oder des zweiten Kraftübertragungsglieds (34) und/oder des ersten Werkzeugelements (20) vorgesehen ist.

17. Instrument nach einem der voranstehenden Ansprüche, **dadurch gekennzeichnet, daß** das Werkzeug ein zweites, relativ zum Schaft (12) feststehendes Werkzeugelement (14) umfaßt.

## Claims

1. A surgical instrument (10) having a shaft (12), a first implement arranged on the distal end of the shaft (12) and comprising an implement part (20) mounted so as to be pivotable about a pivoting axis (16), and a first force transmission member (36, 37) for pivoting the first implement part (20) from a first pivoted position into a second pivoted position, wherein a first control cam (52, 53; 55, 56) is provided for converting a movement of the first force transmission member (36, 37) into the pivoting movement of the first implement part (20) and wherein the first control cam (52, 53; 55, 56) has a shape which converts a force, exertable on the first implement part (20) by the first force transmission member (36, 37), into a turning moment which increases during the pivoting movement, wherein the first control cam comprises two control surfaces (52, 53; 55, 56) sliding along one another, and wherein one of the two control surfaces (52, 53) is a front edge (52, 53) of the first force transmission member (36, 37), and the other (55, 56) is associated with the first implement part (20), **characterised in that** a second force transmission member (34) is provided for pivoting the first implement part (20) back from the second pivoted position into the first pivoted position, **in that** a second control cam (58, 60) is provided for converting a movement of the second force transmission member (34) into a pivoting movement of the first implement part (20) from the second implement-part position into the first implement-part position, and **in that** the control cam (58, 60) has a shape which converts a force, exertable on the first implement part (20) by the second force transmission member (34), into a turning moment which increases during the pivoting movement.

2. An instrument according to claim 1, **characterised in that** the first control cam (52, 53; 55, 56) has a shape which extends the effective lever arm (72) acting on the first implement part (20) during the pivoting movement from the first pivoted position into the second pivoted position.

3. An instrument according to either one of claims 1 and 2, **characterised in that** the control surfaces comprise two substantially flat surfaces (52, 53; 55, 56), and **in that** the control surface (52, 53) associated with the first force transmission member (36, 37) is inclined from the pivoting axis towards the proximal end of the instrument (10).

4. An instrument according to any one of the preceding claims, **characterised in that** the control surfaces (52, 53; 55, 56) abut one another during the movement of the first implement part (20) from the first implement-part position into the second implement-part position.

5. An instrument according to claim 5, **characterised in that** the control surfaces (52, 53; 55, 56) abut one another at a first contact or application point (76).

6. An instrument according to any one of the preceding claims, **characterised in that** the second control cam comprises two return-movement control surfaces (58, 60) sliding along one another, and **in that** one of the two return-movement control surfaces (58) is associated with the second force transmission member (34) and the other (60) is associated with the first implement part (20).

7. An instrument according to claim 6, **characterised in that** the control surface (58) associated with the second force transmission member (34) is concavely curved in relation to the pivoting axis and **in that** the control surface (60) associated with the first implement part (20) is convexly curved in relation to the pivoting axis.

8. An instrument according to either one of claims 6 and 7, **characterised in that** the return-movement control surfaces (58, 60) abut one another during the movement of the first implement part (20) from the second implement-part position into the first implement-part position.

9. An instrument according to claim 8, **characterised in that** the return-movement control surfaces (58, 60) abut one another at a second contact or application point (66).

10. An instrument according to claim 9, **characterised in that** the first application point (76) and the second application point (66) lie on the same side of a plane containing the rotation axis (16), irrespective of the pivoted position of the first implement part (20).

11. An instrument according to any one of the preceding claims, **characterised in that** the first (36, 37) and the second force transmission member (34) each comprise a thrust member (36, 37; 34) for transmitting a thrust force to the first implement part (20).

12. An instrument according to any one of the preceding claims, **characterised in that** the force transmission members (36, 37; 34) are movable in the longitudinal direction of the shaft (12).

13. An instrument according to any one of the preceding claims, **characterised in that** the force transmission members (36, 37; 34) for moving the first implement part (20) are movable in opposite directions.

14. An instrument according to any one of the preceding claims, **characterised in that** at least one movably mounted actuating member (30), associated with the shaft (12), is provided for moving at least one of the two force transmission members (36, 37; 34), and **in that** the at least one of the two force transmission members (36, 37; 34) is mounted on the at least one actuating member (30).

15. An instrument according to claim 14, **characterised in that** the actuating member (30) is pivotably mounted on the instrument (10).

16. An instrument according to any one of the preceding claims, **characterised in that** at least one stop (62) is provided for limiting the movement of the first (36, 37) and/or the second force transmission member (34) and/or the first implement part (20).

17. An instrument according to any one of the preceding claims, **characterised in that** the implement comprises a second implement part (14) which is fixed in relation to the shaft (12).

## Revendications

1. Instrument chirurgical (10) comportant un corps en tige (12) avec un outil comprenant un premier élément d'outil (20) disposé à l'extrémité distale du corps en tige (12) et monté pivotant autour d'un axe de pivotement (16), et avec un premier organe de transmission de force (36, 37) pour faire pivoter le premier élément d'outil (20) d'une première position de pivotement dans une seconde position de pivotement, une première came de commande (52, 53 ; 55, 56) étant prévue pour la transformation d'un mouvement du premier organe de transmission de force (36, 37) en mouvement de pivotement du premier élément d'outil (20), et la première came de commande (52, 53 ; 55, 56) présentant une forme pour convertir une force, qui peut être exercée par le premier organe de transmission de force sur le premier élément d'outil (20), en un couple croissant lors du mouvement de pivotement, la première came de commande comprenant deux surfaces de commande (52, 53 ; 55, 56) qui glissent l'une le long de l'autre, et l'une (52, 53) des deux surfaces de commande étant un bord frontal (52, 53) du premier organe de transmission de force (36, 37) et l'autre (55, 56) étant associée au premier élément d'outil (20),
**caractérisé en ce qu'**il est prévu un second organe de transmission de force (34) pour le pivotement en retour du premier élément d'outil (20) de la seconde position de pivotement à la première position de pivotement, **en ce qu'**il est prévu une seconde came de commande (58, 60) pour la transformation d'un mouvement du second organe de transmission de force (34) en un mouvement de pivotement du premier élément d'outil (20) de la seconde position d'élément d'outil à la première position d'élément d'outil, et **en ce que** la came de commande (58, 60) présente une forme pour convertir une force, qui peut être exercée par le second organe de transmission de force (34) sur le premier élément d'outil (20), en un couple croissant lors du mouvement de pivotement.

2. Instrument selon la revendication 1, **caractérisé en ce que** la première came de commande (52, 53 ; 55, 56) présente une forme pour allonger le bras de levier effectif (72) agissant sur le premier élément d'outil (20) lors du mouvement de pivotement de la première position de pivotement vers la seconde position de pivotement.

3. Instrument selon l'une des revendications 1 ou 2, **caractérisé en ce que** les surfaces de commande englobent deux surfaces (52, 53 ; 55, 56) sensiblement planes, et **en ce que** la surface de commande (52, 53) associée au premier organe de transmission de force est inclinée de l'axe de pivotement en direction de l'extrémité proximale de l'instrument (10).

4. Instrument selon l'une des revendications précédentes, **caractérisé en ce que** les surfaces de commande (52, 53 ; 55, 56) s'appuient l'une sur l'autre pendant le mouvement de l'élément d'outil (20) de la première position d'élément d'outil vers la seconde position d'élément d'outil.

5. Instrument selon la revendication 4, **caractérisé en ce que** les surfaces de commande (52, 53 ; 55, 56) s'appuient l'une sur l'autre au niveau d'un premier point de contact ou d'action (76).

6. Instrument selon l'une des revendications précédentes, **caractérisé en ce que** la seconde came de commande comprend deux surfaces de commande de mouvement de retour (58, 60) glissant l'une le long de l'autre, et **en ce que** l'une (58) des deux surfaces de commande de mouvement de retour est associée au second organe de transmission de force (34) et l'autre (60) au premier élément d'outil (20).

7. Instrument selon la revendication 6, **caractérisé en ce que** la surface de commande (58) associée au second organe de transmission de force (34) présente une courbure concave par rapport à l'axe de pivotement, et **en ce que** la surface de commande (60) associée au premier élément d'outil (20) présente une courbure convexe par rapport à l'axe de pivotement.

8. Instrument selon l'une des revendications 6 ou 7, **caractérisé en ce que** les surfaces de commande de mouvement de retour (58, 60) s'appuient l'une sur l'autre pendant le mouvement du premier élément d'outil (20) de la seconde position d'élément d'outil vers la première position d'élément d'outil.

9. Instrument selon la revendication 8, **caractérisé en ce que** les surfaces de commande de mouvement de retour (58, 60) s'appuient l'une sur l'autre au niveau d'un second point de contact ou d'action (66).

10. Instrument selon la revendication 9, **caractérisé en ce que** le premier point d'action (76) et le second point d'action (66) se situent, indépendamment d'une position de pivotement du premier élément d'outil (20), sur le même côté d'un plan contenant l'axe de rotation (16).

11. Instrument selon l'une des revendications précédentes, **caractérisé en ce que** le premier (36, 37) et le second organe de transmission de force (34) comprennent chacun un organe de poussée (36, 37 ; 34) pour transmettre une force de poussée au premier élément d'outil (20).

12. Instrument selon l'une des revendications précédentes, **caractérisé en ce que** les organes de transmission de force (36, 37 ; 34) sont mobiles dans la direction longitudinale du corps en tige (12).

13. Instrument selon l'une des revendications précédentes, **caractérisé en ce que** les organes de transmission de force (36, 37 ; 34) sont mobiles selon des sens opposés pour déplacer le premier élément d'outil (20).

14. Instrument selon l'une des revendications précédentes, **caractérisé en ce qu'**il est prévu au moins un organe d'actionnement (30) monté mobile et associé au corps en tige (12), pour déplacer au moins l'un des deux organes de transmission de force (36, 37 ; 34), et **en ce qu'**au moins l'un des deux organes de transmission de force (36, 37 ; 34) est monté sur ledit au moins un organe d'actionnement (30).

15. Instrument selon la revendication 14, **caractérisé en ce que** l'organe d'actionnement (30) est monté pivotant sur l'instrument (10).

16. Instrument selon l'une des revendications précédentes, **caractérisé en ce qu'**il est prévu au moins une butée (62) pour limiter le mouvement du premier (36, 37) et/ou du second organe de transmission de force (34) et/ou du premier élément d'outil (20).

17. Instrument selon l'une des revendications précédentes, **caractérisé en ce que** l'outil comprend un second élément d'outil (14) en position fixe par rapport au corps en tige (12).
